# EUROPEAN PATENT APPLICATION

(11) **EP 3 692 888 A1**
(43) Date of publication of application: **12.08.2020**
(21) Application number: 19155928.5
(22) Date of filing: 07.02.2019
(51) Int. Cl.: A61B 1/06, A61B 1/07, A61B 1/24, A61B 5/00

(54) **LIGHT ENGINE CONFIGURED TO BE USED IN AN ORAL EXAMINATION DEVICE**

(71) Applicant: Koninklijke Philips N.V., 5656 AG Eindhoven (NL)
(72) Inventor: RONDA, Cornelis Reinder, 5656 AE Eindhoven (NL); VERMEULEN, Olaf Thomas Johan Antonie, 5656 AE Eindhoven (NL); DEANE, Steven Charles, 5656 AE Eindhoven (NL); OUWERKERK, Martin, 5656 AE Eindhoven (NL)
(74) Representative: de Haan, Poul Erik

(57) **Abstract**

In the context of light-based oral examination, a light engine (1) involving only minimal light losses is provided. The light engine (1) includes a light supply unit (2) for supplying light to be conveyed to a subject's oral cavity, which light supply unit (2) comprises a light source (10) configured to generate light, a luminescent converter (20) configured and arranged to receive light from the light source (10), to emit light to be conveyed to a subject's oral cavity in response to receiving the light from the light source (10), and a light guide (30) configured and arranged to receive light from the luminescent converter (20) and to convey the light to a subject's oral cavity. In a preferred embodiment of the light engine (1), semiconductor quantum dots are associated with at least one surface area of the luminescent converter (1).

## Description

### FIELD OF THE INVENTION

The invention relates to a light engine configured to be used in an oral examination device, the light engine including a light supply unit for supplying light to be conveyed to a subject's oral cavity.

The invention also relates to an oral examination device configured to be at least partially positioned in a subject's oral cavity for examination purposes, the oral examination device comprising a light engine as mentioned, and further relates to an oral examination system comprising such an oral examination device.

### BACKGROUND OF THE INVENTION

Examination of the oral cavity is done for various purposes, including the purpose of detecting gingivitis. In many cases in which a subject's oral cavity is inspected by a person such as a dental professional, use is made of a device which is configured to shine light in the oral cavity, and which is held close to the oral cavity or is at least partially inserted into the oral cavity. In the first place, in such cases, the light may be visible light having a function in ensuring that the oral cavity or at least a portion thereof is brightly lit so that the person may have a clear view on the teeth, the gums and/or other oral features to be inspected. Additionally, or alternatively, the light may have a function in the operation of a spectral analysis system, i.e. a system which is designed to detect at least one characteristic of oral features by irradiating the oral features with light having a certain intensity-wavelength relation and determining if changes of the intensity-wavelength relation are found in reflected light received back from the oral features, wherein such changes are taken as a measure of the characteristic.

In the following, it is assumed that the subject of which the oral cavity is examined is a human being, which does not alter the fact that the term "subject" is to be understood so as to cover animals, particularly animals having teeth and gums, as well.

Gingivitis is a well-known disease, which is encountered in more than half of the adult population. Gingivitis manifests itself by bleeding gums, especially during brushing. Gingivitis is known to be reversible, and it is important that action is taken so as to avoid gingivitis from getting more severe, as gingivitis may eventually develop into periodontitis. In view of the fact that periodontitis can cause irreversible damage to the jaw bone, can result in loss of teeth, and can even result in systemic health issues, there is a need for determining whether a person suffers from gingivitis, so that if this is found to be the case, the person can be advised on improved oral hygiene routines.

It has been found that gingivitis can be detected optically already before bleeding sets in, enabling very early treatment of the disease. According to a sophisticated possibility, reflection of light at a number of different wavelengths is measured, to infer the amount of oxygenated and non-oxygenated hemoglobin. Inflamed tissue has higher relative concentrations of non-oxygenated hemoglobin. A device has been developed in which light from a Light Emitting Diode (LED) is coupled into a light guide, wherein the light is transported to the gums by this light guide, and wherein reflected light is collected by another light guide. It is desirable to use commonly available LEDs and commonly available detectors such as photodiodes in the device so that costs may be as low as possible. However, it is found that operating a spectral analysis system with relatively cheap components involves high loss of light, i.e. a very inefficient use of the light emitted by the LEDs.

### SUMMARY OF THE INVENTION

It is an object of the invention to provide a light engine which is configured to be used in an oral examination device, which includes a light supply unit for supplying light to be conveyed to a subject's oral cavity, and which involves a high light output without a need for having expensive components in the light supply unit. A high light output is favorable because cheap photodetectors or the like are only capable of yielding reliable detection results when the detected light has sufficient intensity. In view thereof, the invention provides a light engine configured to be used in an oral examination device, the light engine including a light supply unit for supplying light to be conveyed to a subject's oral cavity, the light supply unit comprising a light source configured to generate light, a luminescent converter configured and arranged to receive light from the light source, to emit light to be conveyed to a subject's oral cavity in response to receiving the light from the light source, and to realize a shift of the peak wavelength of the received light in the process, and a light guide configured and arranged to receive light from the luminescent converter and to convey the light to a subject's oral cavity.

According to the invention, a light supply unit of a light engine comprises a luminescent converter besides a light source and a light guide. In a practical embodiment, the light source comprises at least one LED. The luminescent converter can easily be designed so as to be larger or smaller, so that the solution according to the invention is easily scalable in the sense that more LEDs may be included in the light engine's design in case this appears to be desirable in order to achieve a certain light intensity. In any case, the size and shape of the luminescent converter can easily be chosen such as to enable the luminescent converter to receive most if not all of the light emitted by the light source, wherein the luminescent converter is designed with a light-incoupling surface area of appropriate size and texture. Further, the luminescent converter can easily be configured so as to outcouple light through only a small, well-defined area, so that loss of light at the interface of the luminescent converter and the light guide can be avoided. In a general sense, when the invention is applied, optical coupling efficiency of a light engine including a light supply unit comprising a light guide is improved because the light guide is coupled to a luminescent converter, rather than directly to a light source.

Preferably, the light source is mounted on the luminescent converter, so that no additional means are needed for mounting the light source, and it is possible for the luminescent converter to receive light shining from the light source directly from the light source. Hence, the preferred option according to which the light source is mounted on the luminescent converter is a factor in having optimal conveyance of light from the light source to the luminescent converter and thereby in having minimal loss of light.

As mentioned earlier, the light source may comprise at least one LED. In case the light source comprises a plurality of LEDs, the LEDs may be positioned with respect to the luminescent converter in any configuration as desired. For example, in such a case, it is not necessary for all of the LEDs to be positioned at one and the same side of the luminescent converter. A plurality of LEDs may be provided as one or more arrays of LEDs.

It may be practical for the light guide to comprise at least one optical fiber.

It may be practical for the luminescent converter to comprise a ceramic material. For example, the luminescent converter may comprise a transparent ceramic material such as a Ce doped garnet material. Additionally, or alternatively, it is also possible for the luminescent converter to comprise one or more other types of material, such as a single crystalline material, a glassy material, or a transparent polymer containing an organic dye.

In an advantageous embodiment of the light engine according to the invention, the luminescent converter is shaped as a block of material having a surface area which is provided with a light-outcoupling texture. In general, the luminescent converter may be a compact, solid piece of material. For example, the luminescent converter may be shaped like a solid beam having a square or rectangular cross-section. Alternatively, the luminescent converter may be shaped as a cylinder having a circular or elliptical cross-section and planar end surfaces.

The block of material as mentioned may have a tapered shape so that a collimation effect can be obtained. In such a case, it is advantageous if the light guide extends from a side of the luminescent converter with respect to which the luminescent converter tapers.

In order to avoid loss of light at the interface of the luminescent converter and the light guide, it is practical and advantageous if the light guide is configured and arranged to receive light from a light-outcoupling surface area of the luminescent converter, a light-incoupling surface area of the light guide being as large as or larger than the light-outcoupling surface area of the luminescent converter. Further, a light reflector covering the luminescent converter with the exception of at least the light-outcoupling surface area of the luminescent converter may be used, so that conveyance of light from the luminescent converter to the light guide may even further be optimized. The light reflector may be arranged so as to cover all surface areas of the luminescent converter outside of the light-outcoupling surface area, but it is also possible that the light reflector is arranged to only cover a surface area surrounding the light-outcoupling surface area, for example.

According to an advantageous option existing within the framework of the invention, semiconductor quantum dots are associated with at least one surface area of the luminescent converter, wherein the semiconductor quantum dots may be arranged on or (directly) adjacent to the at least one surface area in any suitable manner. By having one or more sides of the luminescent converter covered with quantum dots, it is achieved that relatively narrow spectral bands can be obtained, which can be chosen so as to be exactly in the spectral regions needed in the intended use of the light engine. The quantum dots may be arranged on the at least one surface area of the luminescent converter as a film. Further tuning of the color of the light to be emitted by the luminescent converter at the position of the quantum dots can be done by choosing an appropriate composition and size of the quantum dots. The quantum dots may be of substantially the same size or may be of different size, whatever is appropriate in a given situation. In general, the quantum dots may be of one particular type or may be of various types. For the sake of completeness, it is noted that quantum dots are known to be semiconductor particles which are significantly smaller than LEDs, and that many types of quantum dot emit light of specific frequencies, i.e. essentially monochromatic light, if electricity or light is applied to them, wherein these frequencies can be precisely tuned by changing the dot's size, shape and material.

As mentioned earlier, the invention also relates to an oral examination device configured to be at least partially positioned in a subject's oral cavity for examination purposes, the oral examination device comprising a light engine including a light source, a luminescent converter and a light guide as defined and explained in the foregoing, wherein the light engine may be of any suitable embodiment. Such an oral examination device may further comprise a detector unit for receiving light originating from the light engine and reflected in the subject's oral cavity, in which case a processor of a suitable type may be used for processing signals obtained on the basis of the light received by the detector unit and determining one or more characteristics of oral features on the basis thereof. In such a case, the processor may be configured to communicate, preferably in a wireless fashion, with an external device or system which is separate from the oral examination device, and which is configured to provide information to a person, such as a smartphone.

The above-described and other aspects of the invention will be apparent from and elucidated with reference to the following detailed description of a light engine which includes a light supply unit comprising a light source, a luminescent converter and a light guide, which is designed to have high light output, and which is especially suitable for use in an oral examination device.

### BRIEF DESCRIPTION OF THE DRAWINGS

The invention will now be explained in greater detail with reference to the figures, in which equal or similar parts are indicated by the same reference signs, and in which:
Figure 1 illustrates the basics of the design of a light engine according to the invention;
Figure 2 diagrammatically shows a first embodiment of an oral examination device which is equipped with a light engine according to the invention;
Figure 3 diagrammatically shows a second embodiment of an oral examination device which is equipped with a light engine according to the invention; and.
Figure 4 diagrammatically shows a third embodiment of an oral examination device which is equipped with a light engine according to the invention.

### DETAILED DESCRIPTION OF EMBODIMENTS

The invention is explained in the context of examining a subject's oral cavity in order to obtain information which is suitable to be used for assessing the subject's gum health, particularly assessing whether or not it may so that the subject suffers from gingivitis. In the art, spectral analysis systems are known in which light from an LED is coupled into a light guide, wherein the light guide is used to transport the light to a subject's gums, and wherein the systems comprise another light guide for collecting the reflected light. However, in the known systems, large losses are encountered in the light-incoupling process. In the first place, there is a mismatch between the dimensions of commonly available LEDs and the diameter of the light guide. In the second place, there is a mismatch between the light-outcoupling angle of the LED and the light-incoupling angle of the light guide. Further, as far as the detection of light is concerned, it may be so that commonly available photodiodes covered by a bandpass filter are used in order to save costs. However, in such a case, it is achieved that only a small fraction of the total amount of light is allowed to reach the photodiodes, while the rest is not used.

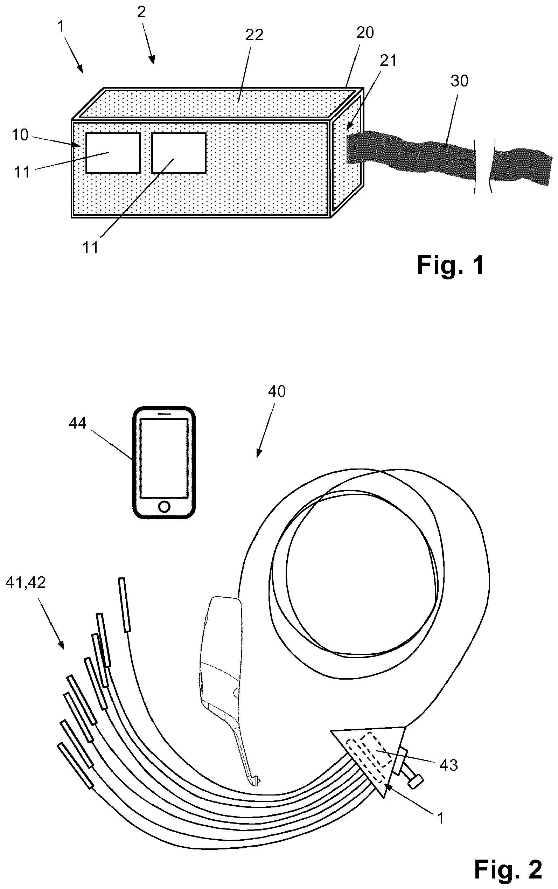

Figure 1 diagrammatically shows a light engine 1 according to the invention. The light engine 1 includes a light supply unit 2 comprising a light source 10, a luminescent converter 20 for receiving light from the light source 10, and a light guide 30 for receiving light from the luminescent converter 20 and conveying the light to a subject's oral cavity (not shown). It is practical for the light source 10 to comprise one or more LEDs 11. In such a case, the luminescent converter 20 may particularly be configured and arranged to change blue light emitted by the one or more LEDs 11 into green-red light, dependent on the composition of the luminescent converter 20. As the emission spectrum depends slightly on the composition of the luminescent converter 20, the emission spectrum can be slightly optimized towards the intended use. In any case, the luminescent converter 20 may be a ceramic light converter or any other suitable type of light converter, such as a light converter comprising single crystalline material, glassy material, or a suitable transparent polymer in which an organic dye is incorporated.

Further, it is practical for the one or more LEDs 11 to be mounted on the luminescent converter 20. An example of a suitable length of the luminescent converter 20 is about 2 cm, wherein it is noted that the luminescent converter 20 can be designed so as to be longer or shorter, as desired in view of a number of LEDs 11 to be used and a light output which needs to be realized.

The light guide 30 is mounted at one side of the luminescent converter 20. The luminescent converter 20 has a light-outcoupling window 21 (light-outcoupling surface area) having a diameter which is equal to or smaller than a diameter of the light guide 30 at the position of the interface of the luminescent converter 20 and the light guide 30. In this way, loss of light is avoided in the transition of the luminescent converter 20 to the light guide 30. The remaining surface area of the luminescent converter 20 may be covered with a light reflector 22 so as to minimize light outcoupling losses. At least when the luminescent converter 20 is a ceramic converter, losses due to light absorption in the luminescent converter 20 are negligible, as the material of the luminescent converter 20 may involve a sufficiently large Stokes shift. In this respect, use of transparent luminescent ceramic garnets is a practical possibility.

It is advantageous to use semiconductor quantum dots with the luminescent converter 20. For example, the side of the luminescent converter 20 where the light guide 30 is present may be partially covered by quantum dot film, namely at the position of the light-outcoupling window 21. A garnet luminescent converter is known to involve a very broad emission spectrum and a rather low emission intensity in the red spectral range. By using quantum dots with the luminescent converter 20, of one or more types, relatively narrow spectral bands can be obtained, also in the red part of the spectrum. This allows for designing the light engine 1 in such a way that spectral bands in spectral regions as envisaged can actually be realized. Further tuning of the color of the light can be done by choosing an appropriate composition and size of the quantum dots. Tuning of the relative intensity of the quantum dot emission can be done by choosing an appropriate size of the surface area of the luminescent converter 20 covered by quantum dots. Examples of quantum dots which may be used in the context of the light engine 1 include InP quantum dots with sizes in the nm range and CdS quantum dots, wherein it is noted that the first option is preferred in view of the fact that CdS quantum dots are poisonous whereas InP quantum dots are not. Another example of useable quantum dots is CdSe quantum dots. In view of the fact that the excitation spectra of quantum dots are very broad, quantum dots are very well suitable to be used with blue light from the one or more LEDs 11 and green light from the luminescent converter 20.

In the light engine 1 according to the invention, all light emitted by the one or more LEDs 11 is used, while the optional application of semiconductor quantum dots results in better utilization of light to be detected. 100% of the light can be made to enter the light guide 30. Assuming a maximum of 50% reflection and absorption losses in the luminescent converter 20, wherein it is noted that the actual losses are expected to be much lower, it is achieved that at least 50% of the light is used. When quantum dots are used to adapt the spectrum of the light, light losses are reduced even further by about 50% or more as part of the emission spectrum is very well adapted to what is needed for detecting light in the oral cavity. In addition, the intensity of deep red light is increased.

As the light engine 1 involves a very efficient use of light, application of the light engine 1 allows for using relatively cheap components for detecting light in the oral cavity. When unexpectedly the light intensity of a certain embodiment of the light engine 1 would appear to still be too low, the number of LEDs 11 can be increased in the design of the light engine 1, wherein the size of the luminescent converter 20 can be adapted in the design of the light engine 1 if necessary.

Figure 2 relates to a first embodiment of an oral examination device 40 which is equipped with a light engine 1 according to the invention. The oral examination device 40 comprises a number of optical fibers 41 for transporting light to a subject's gums and a number of optical fibers 42 for transporting light from a subject's gums, the first optical fibers 41 constituting the light guide 30 of the light engine 1. Processing of the light detected inside the subject's oral cavity is done by a processor 43 which may be part of an external device or may be incorporated in the oral examination device 40, as shown. In any case, it is advantageous if detection results are communicated to a smartphone 44, tablet or the like so that a person such as a dentist or other dental professional may be informed about those results. Such communication can take place at a long range such as through the Internet, or at a significantly shorter range.

The oral examination device 40 may comprise a battery for providing the power which is necessary for driving the light engine 1. According to another possibility, the oral examination device 40 may be connectable to the grid, which is especially advantageous when a plurality of LEDs 11 is included in the light engine 1.

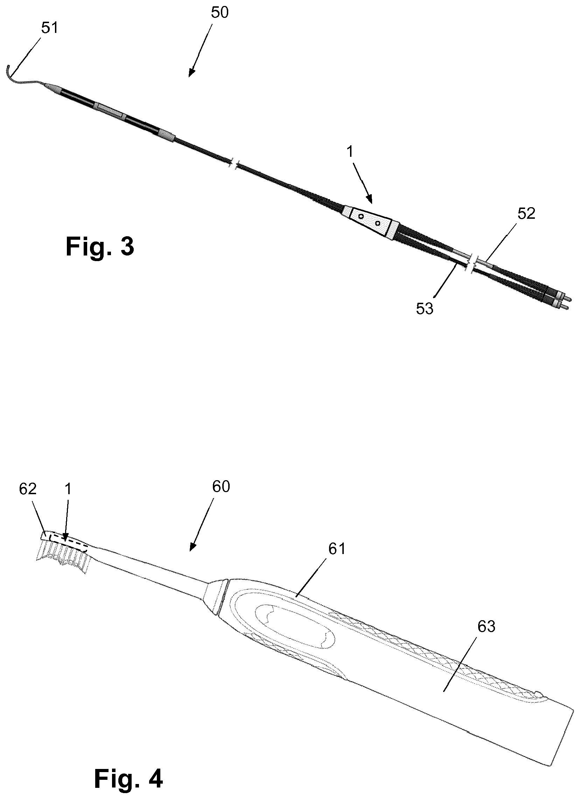

The light engine 1 according to the invention is suitable for use with many different types of dental tools. Figure 3 relates to a second embodiment of an oral examination device 50 which is equipped with a light engine 1 according to the invention, which comprises a regular hook-like tool 51 for measuring gingivitis by probing the gums and detecting whether the gums bleed as a result thereof. In the shown example, the oral examination device 50 comprises the light engine 1 having an optical fiber 52 for transporting light to a subject's gums, and further comprises an optical fiber 53 for transporting light from a subject's gums.

Within the framework of the invention, an oral examination device is feasible which does not only have a light-based oral examination function, but which has at least one other additional function. One example of such an oral examination device is illustrated in figure 4. The oral examination device 60 shown in figure 4 comprises a power toothbrush 61 incorporating the light engine 1, the additional function being a cleaning function (tooth brushing function). The light engine 1 may be included in the brushhead 62 of the toothbrush 61, as shown, but may alternatively be included in the handle 63 of the toothbrush 61, for example.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the words "comprising" and "including" do not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention can be summarized as follows. In the context of light-based oral examination, a light engine 1 involving only minimal light losses is provided. The light engine 1 includes a light supply unit 2 for supplying light to be conveyed to a subject's oral cavity, which light supply unit 2 comprises a light source 10 configured to generate light, a luminescent converter 20 configured and arranged to receive light from the light source 10, to emit light to be conveyed to a subject's oral cavity in response to receiving the light from the light source 10, and a light guide 30, 41, 52 configured and arranged to receive light from the luminescent converter 20 and to convey the light to a subject's oral cavity. In a preferred embodiment of the light engine 1, semiconductor quantum dots are associated with at least one surface area of the luminescent converter 1.

## Claims

1. Light engine (1) configured to be used in an oral examination device (40, 50, 60), the light engine (1) including a light supply unit (2) for supplying light to be conveyed to a subject's oral cavity, the light supply unit (2) comprising
- a light source (10) configured to generate light,
- a luminescent converter (20) configured and arranged to receive light from the light source (10), to emit light to be conveyed to a subject's oral cavity in response to receiving the light from the light source (10), and to realize a shift of the peak wavelength of the received light in the process, and
- a light guide (30, 41, 52) configured and arranged to receive light from the luminescent converter (20) and to convey the light to a subject's oral cavity.

2. Light engine (1) according to claim 1, wherein the light source (10) is mounted on the luminescent converter (20).

3. Light engine (1) according to claim 1 or 2, wherein the light source (10) comprises at least one Light Emitting Diode (LED) (11).

4. Light engine (1) according to any of claims 1-3, wherein the light guide (30, 41, 52) comprises at least one optical fiber.

5. Light engine (1) according to any of claims 1-4, wherein the luminescent converter (20) comprises at least one of a ceramic material, a single crystalline material, a glassy material, and a transparent polymer in which an organic dye is incorporated.

6. Light engine (1) according to any of claims 1-5, wherein the luminescent converter (20) is shaped as a compact, solid piece of material having a surface area which is provided with a light-outcoupling texture.

7. Light engine (1) according to any of claims 1-6, wherein the luminescent converter (20) is shaped as a cylinder having a circular or elliptical cross-section and planar end surfaces.

8. Light engine (1) according to any of claims 1-7, wherein the light guide (30, 41, 52) is configured and arranged to receive light from a light-outcoupling surface area of the luminescent converter (20), a light-incoupling surface area of the light guide (30, 41, 52) being as large as or larger than the light-outcoupling surface area of the luminescent converter (20).

9. Light engine (1) according to claim 8, comprising a light reflector (22) covering the luminescent converter (20) with the exception of at least the light-outcoupling surface area of the luminescent converter (20).

10. Light engine (1) according to any of claims 1-9, wherein semiconductor quantum dots are associated with at least one surface area of the luminescent converter (20).

11. Light engine (1) according to claim 10, wherein the quantum dots are arranged on the at least one surface area of the luminescent converter (20) as a film.

12. Oral examination device (40, 50, 60) configured to be at least partially positioned in a subject's oral cavity for examination purposes, the oral examination device (40, 50, 60) comprising a light engine (1) according to any of claims 1-11.

13. Oral examination device (40, 50, 60) according to claim 12, further comprising a detector unit configured and arranged to receive light originating from the light engine (1) and reflected in the subject's oral cavity.

14. Oral examination system, comprising an oral examination device (40, 50, 60) according to claim 13 and a processor (43) configured and arranged to process signals obtained on the basis of the light received by the detector unit of the oral examination device (40, 50, 60) and to determine at least one characteristic of oral features on the basis thereof.

15. Oral examination system according to claim 14, wherein the processor (43) is configured and arranged to communicate with an external device or system (44) which is separate from the oral examination device (40, 50, 60), and which is configured to provide information to a person.
